(19) Europäisches Patentamt
European Patent Office
Office européen des brevets

(11) **EP 1 874 140 B1**

(12) **EUROPEAN PATENT SPECIFICATION**

(45) Date of publication and mention of the grant of the patent:
**09.02.2011 Bulletin 2011/06**

(21) Application number: **06724199.2**

(22) Date of filing: **31.03.2006**

(51) Int Cl.:
*A23L 1/305* (2006.01)     *A23C 9/13* (2006.01)
*A61K 38/01* (2006.01)     *A61K 38/06* (2006.01)
*A23L 1/304* (2006.01)     *A61P 9/12* (2006.01)
*A61P 3/04* (2006.01)     *A61P 3/06* (2006.01)

(86) International application number:
**PCT/EP2006/003265**

(87) International publication number:
**WO 2006/114194 (02.11.2006 Gazette 2006/44)**

(54) **PEPTIDES HAVING A HEALTH BENEFIT AND COMPOSITIONS COMPRISING THEM**

GESUNDHEITSFÖRDERNDE PEPTIDE UND SIE ENTHALTENDE ZUSAMMENSETZUNGEN

PEPTIDES PRESENTANT UN EFFET BENEFIQUE POUR LA SANTE ET COMPOSITIONS CONTENANT CES PEPTIDES

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.04.2005 EP 05076014**

(43) Date of publication of application:
**09.01.2008 Bulletin 2008/02**

(73) Proprietors:
• **Unilever N.V.**
**3013 AL Rotterdam (NL)**
Designated Contracting States:
**AT BE BG CH CZ DE DK EE ES FI FR GR HU IS IT LI LT LU LV MC NL PL PT RO SE SI SK TR**
• **Unilever PLC**
**London**
**EC4Y 0DY (GB)**
Designated Contracting States:
**CY GB IE**

(72) Inventors:
• **GERHARDT, C.C.,**
**Unilever R & D Vlaardingen**
**NL-3133 AT Vlaardingen (NL)**
• **VAN PLATERINK, C.J.,**
**Unilever R & D Vlaardingen**
**NL-3133 AT Vlaardingen (NL)**
• **EDENS, Luppo**
**NL-3062 JL Rotterdam (NL)**
• **DE ROOS, Andre, L.**
**NL-2614 TB Delft (NL)**

(74) Representative: **Corsten, Michael Allan et al**
**Unilever Patent Group**
**Olivier van Noortlaan 120**
**3133 AT Vlaardingen (NL)**

(56) References cited:
**WO-A-2004/082709     WO-A-2005/012334**
**CN-A- 1 552 891     US-A1- 2002 182 301**

• **YAMAMOTO N ET AL: "ANTIHYPERTENSIVE PEPTIDE DERIVED FROM MILK PROTEINS" DIE NAHRUNG, VCH VERLAGSGESELLSCHAFT, WEINHEIM, vol. 43, no. 3, 1999, pages 159-164, XP008015569 ISSN: 0027-769X**
• **YAMAMOTO N ET AL: "Antihypertensive effect of the peptides derived from casein by an extracellular proteinase from Lactobacillus helveticus CP790" JOURNAL OF DAIRY SCIENCE, AMERICAN DAIRY SCIENCE ASSOCIATION. CHAMPAIGN, ILLINOIS, US, vol. 77, 1994, pages 917-922, XP002095347 ISSN: 0022-0302**
• **MAENO ET AL: "Identification of an antihypertensive peptide from casein hydrolyzate produced by a proteinase from Lactobacillus helveticus CP790" JOURNAL OF DAIRY SCIENCE, AMERICAN DAIRY SCIENCE ASSOCIATION. CHAMPAIGN, ILLINOIS, US, vol. 79, no. 8, 1996, pages 1316-1321, XP002095593 ISSN: 0022-0302**
• **PATENT ABSTRACTS OF JAPAN vol. 013, no. 174 (C-589), 25 April 1989 (1989-04-25) & JP 01 005497 A (KANEBO LTD), 10 January 1989 (1989-01-10)**

- SEPPO L, JAUHIAINEN T, POUSSA T, KORPELA R: "A fermented milk high in bioactive peptides has a blood pressure-lowering effect in hypertensive subjects" AM. J. CLIN. NUTR., vol. 77, 2003, pages 326-330, XP002330912

- YAMAMOTO N ET AL: "PURIFICATION AND CHARACTERIZATION OF AN ANTIHYPERTENSIVE PEPTIDE FROM A YOGURT-LIKE PRODUCT FERMENTED BY LACTOBACILLUS HELVETICUS CPN4" JOURNAL OF DAIRY SCIENCE, AMERICAN DAIRY SCIENCE ASSOCIATION. CHAMPAIGN, ILLINOIS, US, vol. 82, no. 7, July 1999 (1999-07), pages 1388-1393, XP000850204 ISSN: 0022-0302

**EP 1 874 140 B1**

**Description**

**Field of the invention**

The invention relates to certain peptides for use as health benefit agents. Further, the invention relates to certain peptides for use in the areas of the prevention of obesity or body weight control and cardiovascular health maintenance, especially the inhibition of angiotensin-converting enzyme. The invention further relates to food products comprising certain peptides and which are suitable for providing the above-mentioned health-benefits.

**Background to the invention**

In general, people are becoming more aware of the importance of health-issues and are looking for effective and convenient ways to maintain and improve their well-being. This may result in consumers looking for ways of maintaining their present good state of health, or, seeking to improve a particular aspect of their health.

However, whilst consumers often profess a desire to consume a healthy diet and healthy foods, generally they are not very willing to forego convenience and enjoyment to do so. Therefore, generally any product intended to maintain or improve health which is to find wide acceptance amongst consumers will need to be effective in terms of the health-benefit provided, be convenient for the consumer to use and also be acceptable in terms of organoleptic properties. Ideally consumers want to obtain health benefits from predominantly natural ingredients.

As indicated above, consumers have generally shown themselves to be unwilling to compromise on the enjoyment they get from food products in order to obtain a health-benefit therefrom. Therefore, it is also important that the food products and ingredients to be used to provide health benefits are acceptable in terms of organoleptic properties.

The incidence of obesity and the number of people considered overweight in many countries has drastically increased over the last decade. Since obesity and being overweight are generally known to be associated with a variety of diseases such as heart disease, hypertension, type 2 diabetes and arthereosclerosis, this increase is a major health concern for the medical world and for individuals alike. Furthermore, being overweight is considered by the majority of the Western population as unattractive.

This has led to an increasing interest by consumers in maintenance or reduction of their body weight and has created a demand for products that can be used by consumers for these purposes. Especially of interest to consumers are such food products which can conveniently be consumed as part of their daily diet. Examples of such products includes meal replacer products, such as meal replacement bars and beverages. These meal replacer products are generally intended to be consumed as a single-serving food product, such as a bar or a beverage, to replace one or two meals per day.

However a general problem with foods intended to be used to maintain or reduce body weight is that whilst they provide a controlled number of calories per serving, the consumer often feels that the satiety effect obtained from these products is not optimal. This may result in the consumer experiencing hunger feelings in a relatively short time after eating the products, and/or, not feeling fully satiated after eating the products. Both of these scenarios can make it more difficult for a subject to follow a calorie-controlled diet or other weight management plan.

Hypertension or high blood pressure is considered to be one of the main risk factors for Cardio Vascular Diseases (CVD). One of the mechanisms which regulates blood pressure is the renin-angiotensin system. This is a cascade of reactions leading to the formation of angiotensin II, which has a strong vasoconstrictive, and hence blood pressure increasing, effect. Inhibition of one of the key enzymes in this cascade: Angiotensin I Converting Enzyme (ACE) reduces formation of angiotensin II and thus has a blood pressure lowering effect. Long term human intervention studies have shown regular intake of low amounts of ACE inhibitors reduces CVD by 25% (Gerstein et al. (2000), The Lancet 355, 253-259).

ACE-inhibitors in food products are well known. Such food products have for instance been prepared by fermentation of milk or milk products. In a placebo-controlled study, the blood pressure lowering effect of VPP and IPP in sour milk was shown in hypertensive humans (Hata, Y et al. (1996), American Journal of Clinical Nutrition 64, 767-771).

Yamamoto N et al., Die Nahrung, vol. 43, no. 3, (1999), 159-164 provide a review of angiotensin I- converting enzyme inhibitory peptides originating from milk proteins. Tripeptides mentioned in this article include PLW, VAP, IPP and VPP.

Yamamoto N et al., J. of Dairy Sci., vol. 77, (1994), 917-922 describe the results of a study into the antihypertensive effect of peptides derived from casein by an extracellular protease from *Lactobacillus helveticus* CP790. The ACE-inhibiting activity of the 25 main peptides isolated from the casein hydrolysate was determined. The peptides included in this analysis each comprise 4 or more amino acid residues.

Maeno et al., J. of Dairy Sci., vol. 79, no. 8, (1996), 1316-1321 describe the identification of an antihypertensive peptide from casein hydrolysate produced by a protease from *Lactobacillus helveticus* CP790. The authors describe the isolation of 10 peptides from the aforementioned casein hydrolysate. These peptides each comprise 4 or more amino acid residues.

Post-published patent application EP 1 690 869 relates to the production and use of functional foods and other types of foods, comprising the MAP peptide, and having a preventive action on hypertension.

A commercially available fermented milk product, which claims to be "suitable for those with mild hypertension" is Calpis sour milk, fermented with *Lactobacillus helveticus* and *Saccharomyces cervisiae*, produced by Calpis Food Industry, Japan. Another commercially available fermented milk product is Evolus produced by Valio, Finland. These fermented milk products are fermented with *Lactobacillus helveticus* (*Lb.*

*helveticus*) strains. The products contain bio-active peptides (VPP and IPP) which are produced by proteolysis of caseins and which showed *in vitro* ACE inhibition.

However, despite the above developments, there is still a need to provide natural ingredients which can safely be either be consumed on their own, or incorporated into various food products, and which provide one or more health benefits to the subject consuming the ingredient or food product. Furthermore, there is a need to provide such food products which are not adversely affected by the inclusion of such ingredients e.g. in terms of stability and/or organoleptic properties. Consumers have not shown to date a great willingness to compromise on the taste and/or quality of their food whilst still desiring to obtain health benefits from their diets.

It is thus an object of the present invention to address one or more of the above problems.

It is a further object of the invention to provide an edible material which can be used to provide health benefits to a subject consuming it. It is yet a further object to provide such an edible material which can conveniently be ingested either in isolated form or incorporated into a food product.

It is a further object of the invention to provide a food product, or an ingredient which can be incorporated therein, which is suitable for use in body weight control programmes.

It is a further object of the invention to provide a food product, or an ingredient which can be incorporated therein, which is suitable for helping to maintain cardiovascular health, e.g. through ACE inhibition.

It is a further object of the invention to provide a food product, or an ingredient which can be incorporated therein, which have acceptable stability and/or organoleptic properties, in particular good taste, such as an absence of or an acceptable level of bitterness.

It is a further object to provide a food product having a high concentration of an ingredient which provides a health benefit, such as aiding the prevention of obesity/ body weight control and/or helping maintain cardiovascular health, wherein the cardiovascular health maintenance comprises the control of blood cholesterol levels.

**Summary of the invention**

The invention is defined by the claims.

Surprisingly, one or more of these objects is attained according to the invention by the use of the tripeptide MAP and/or salts thereof either in isolated form, or, for the preparation of a food product which provides a health benefit upon consumption.

According to a first aspect the present invention provides the use of the tripeptide MAP and/or salts thereof for the manufacture of a functional food product for the prevention of obesity or body weight control.

According to a second aspect the present invention provides the use of the tripeptide MAP and/or salts thereof for the manufacture of a functional food product for cardiovascular health maintenance, wherein the cardiovascular health maintenance comprises the control of blood cholesterol levels .

It is especially preferred according to the present invention that cardiovascular health maintenance comprises the inhibition of angiotensin-converting (ACE) enzyme and/or the control of blood glucose levels.

According to a third aspect the present invention provides a functional food product capable of providing a health benefit to the consumer thereof, said health benefit selected from the prevention of obesity, body weight control and cardiovascular health maintenance and comprising an amount of 0.1 mg/kg or more of MAP.

According to a fourth aspect the present invention provides a process for preparing a functional food product, the process comprising the steps:

(a) enzymatic hydrolysis of a casein protein substrate comprising beta-casein and/or alpha-s2-casein resulting in a hydrolysed casein product;
(b) separation from the hydrolysed casein product of a fraction rich in tripeptide MAP;
and
(c) using the fraction rich in tripeptide MAP as an ingredient in the preparation of the functional food product, wherein the fraction rich in tripeptide MAP from step b) is dried to obtain a solid rich in tripeptide MAP and the solid is used in step c) as an ingredient in the preparation of the functional food product.

One of the advantages of the tripeptides according to the present invention is that they have been found to be relatively stable in the gastro-intestinal tract. This allows for good activity of the tripeptides after consumption so that effective health benefits may be obtained therefrom after consumption.

A further advantage of the tripeptides according to the present invention is that they can be conveniently incorporated into food products, to produce, functional food products, without unacceptably affecting the stability and/or organoleptic properties thereof.

"Health benefit agent(s)" according to the present invention are materials which provide a health benefit, that is which have a positive effect on an aspect of health or which help to maintain an aspect of good health, when ingested, these aspects of good health being prevention of obesity, body weight control and cardiovascular health maintenance. "Health benefit" means having a positive effect on an aspect of health or helping to maintain an aspect of good health.

"Functional food products" according to the present invention are defined as food products (including for the avoidance of doubt, beverages), suitable for human consumption, in which MAP and/or ITP is used as an ingredient in an effective amount, such that a noticeable health benefit for the consumer of the food product is obtained.

The term "comprising" where used herein is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material or conditions of reaction, physical properties of materials and/or use are to be understood as modified by the word "about." All amounts are by weight, based on the total weight of the relevant product, unless otherwise specified.

For a more complete explanation of the above and other features and advantages of the invention, reference should be made to the following description of the preferred embodiments. Unless otherwise stated, the preferred embodiments apply to all aspects of the invention and can be used as appropriate for each aspect.

## Detailed description of the invention

MAP (Met-Ala-Pro) corresponds to beta-casein position 102-104, and ITP (Ile-Thr-Pro) to alpha-s2-casein position 119-121.

According to the present invention the tripeptide MAP may be used to provide a range of health benefits to a subject consuming them (either in isolated form in incorporated into a food product to provide a functional food product. In particular, the health benefits which may be provided by the use of MAP include;

- the prevention of obesity / body weight control,
- the maintenance of cardiovascular health, through the control of blood cholesterol levels.

### Production of MAP

MAP may be made by hydrolysis or fermentation of any protein substrate containing the amino acid sequence MAP. Advantageously the protein substrate contains both amino acid sequences MAP and ITP.

Through optimisation of the fermentation or hydrolysis conditions, the production of the biologically active molecules MAP and/or ITP. may be maximised. The skilled person trying to maximise the production will know how to adjust the process parameters, such as hydrolysis time, hydrolysis temperature, enzyme type and concentration etc.

For hydrolysate-optimisation, the identity of the precursors of the active peptides needs to be known. However, detection and identification of the biologically active peptides in complex hydrolysates or ferments is a challenging task. Typically, just a few biologically active peptides are present at relatively low levels in a complex sample containing thousands of peptides. Traditional identification approaches employing repeated cycles of high-performance liquid chromatographic (HPLC) fractionation and biochemical evaluation are generally time consuming and prone to losses of activity, and thus are not fully satisfactory.

In the present work a continuous flow biochemical assay is coupled on-line to an HPLC fractionation system. For the determination of the ACE-inhibition characteristics of MAP and ITP, the HPLC column effluent is split between a continuous flow ACE bioassay and a chemical analysis technique (mass spectrometry). Crude hydrolysates are separated by HPLC, after which the presence of biologically active compounds is detected by means of the on-line biochemical assay. Mass spectra are recorded continuously. Hence, structural information is immediately available when a peptide shows a positive signal on the biochemical assay. Other continuous flow biochemical assays may suitably be used to assess other potential biological activities of the peptides.

Health benefit agent

MAP and ITP may be used directly, in isolated form or as part of a peptide mixture, to provide the health benefits described herein to the consumer thereof. However, it is preferred according the present invention that MAP and ITP are incorporated into food products to provide functional food products which provide the health benefits described herein to the consumers of these food functional food products.

Functional food products; production

Functional food products according to the present invention provide a noticeable health benefit for the consumer of the food product. In particular, the noticeable health effect is preferably at least one of the following; body weight reduction or body weight maintenance, appetite suppression or the provision of satiety and/or the maintenance of cardiovascular health including the inhibition of angiotensin-converting enzyme.
The terms "functional food product(s)" and "food product (s)" are used interchangeably herein where both refer to food products comprising the tripeptides of the invention.
The food products according to the invention may be made by any suitable process. Preferably, they are made a process comprising the following steps:

(a)   enzymatic hydrolysis of a casein protein substrate comprising beta-casein and/or alpha-s2-casein resulting in a hydrolysed casein product;
(b)   separation from the hydrolysed casein product of a fraction rich in tripeptide MAP; and
(c)   using the fraction rich in tripeptide MAP as an ingredient in the preparation of the functional food product.

It is especially preferred that the functional food products are made according to a process comprising the following steps:

(a)   enzymatic hydrolysis of a protein substrate comprising hydrolysed protein product;
(b)   separation from the hydrolysed protein product of a fraction rich in tripeptide MAP; and optionally
(c)   concentrating and/or drying the fraction from step b) to obtain a solid rich in tripeptide MAP; and
(d)   using the solid prepared in step c) as an ingredient in the preparation of the food product.

The enzymatic hydrolysis step (a) may be any enzymatic treatment of a suitable protein substrate leading to hydrolysis of the protein resulting in liberation of MAP.
Preferably the protein substrate may be any material that contains the amino acid sequence MAP. Protein substrates known to encompass MAP are, for example, casein, wheat gluten, isolate, egg protein, rice protein, and sunflower protein. Examples of especially suitable substrates include whole milk, skimmed milk, (acid) casein or caseinate, rennet casein, acid whey products or cheese whey products,.
Most preferably the protein substrate is casein or milk. milk, casein, casein powder, casein powder concentrates, casein powder isolates, or beta-casein, or alpha-s2-casein. Preferably a substrate that has a high content of casein, such as casein protein isolate (CPI) or caseinate.
The enzyme used in step (a) may be any enzyme that is able of hydrolysing beta-casein and/or alpha-s2-casein resulting in the liberation of MAP. A suitable hydrolysate containing MAP and ITP may be obtained by hydrolysis with an endo-protease and a tri-peptidase as described in WO03/102905
The separation step (b) (or concentration step (b)) may be executed in any way known to the skilled person, e.g. by filtration, centrifugation or chromatography and combinations thereof. Preferably the separation step (b) is executed using ultrafiltration (UF) and/or nanofiltration (NF) techniques. The pore size of the membranes used in the filtration step, as well as the charge of the membrane may be used to control the separation of the tripeptide MAP. The fractionation of casein protein hydrolysates using charged UF/NF membranes is described in Y. Poilot et al, Journal of Membrane Science 158 (1999) 105-114. Electrodialysis is for instance described in WO00/42066.
The drying step (c) involves drying the fraction from step b) to obtain a solid rich in tripeptide MAP.
This step may be done in a conventional way, e.g. by spray drying or freeze drying.
The fraction rich in peptides prepared in step (b) is hereafter designated as Health-benefit-fraction and the solid prepared in step (c) is hereafter designated as Health-benefit-solid. The Health-benefit-fraction and/or the Health-benefit-solid may advantageously be used as a health benefit agent on its own and it may also be used as an ingredient in a functional food product.

Functional food products; ingredients

The food product according to the invention, or food products derived therefrom, may be pasteurised or sterilised. When the tripeptides of the invention are added to food products, a functional food product is provided.

The functional food products according to the invention may be of any food type, including beverages. They may comprise common food ingredients such as flavours, sugars, fruits, minerals, vitamins, stabilisers, thickeners, etc. in appropriate amounts depending upon the type of food product.

Depending upon the health benefit intended to be delivered to the consumer of the functional food product, the functional food product according to the invention preferably comprises 50-200 mmol/kg $K^+$ and/or 15-60 mmol/kg $Ca^{2+}$ and/or 6-25 mmol/kg $Mg^{2+}$. More preferably the food product comprises, 100-150 mmol/kg $K^+$ and/or 30-50 mmol/kg $Ca^{2+}$ and/or 10-25 mmol/kg $Mg^{2+}$, most preferably 110-135 mmol/kg $K^+$ and/or 35-45 mmol/kg $Ca^{2+}$ and/or 13-20 mmol/kg $Mg^{2+}$. These cations have a beneficial health effect, e.g. of further lowering blood pressure, when incorporated in the food products according to the invention.

Advantageously the functional food product comprises one or more B-vitamins, especially when the food product is intended to provide ACE-inhibition effects/protection against cardiovascular disease. The B-vitamin is preferably one or more of folic acid, Vitamin B2, Vitamin B6, and Vitamin B12. Preferably the composition comprises all of the B-vitamins folic acid, Vitamin B2, Vitamin B6, and Vitamin B12.

Folic acid is the synthetic, stable form of naturally occuring folates. Folic acid is known to participate in the metabolism of homocysteine which is an amino acid in the human diet. High homocystein levels have been correlated to an increased risk of cadiovascular disease. It is thought that lowering homocysteine may reduce the risk of cardiovascular disease. Herein the term folic acid also includes folates.

Vitamins B6 and B12 are known to interfere with the biosynthesis of purine and thiamine, to participate in the synthesis of the methyl group in the process of homocysteine methylation for producing methionine and in several growth processes. Vitamin B6 (pyridoxine hydrochloride) is a known vitamin supplement. Vitamin B12 (cyanobalamin) contributes to the health of the nervous system and is involved in the production of red blood cells. It is also known as a vitamin in food supplements.

In view of their combined positive effect on cardiovascular disease risk reduction, it is preferred that functional food products according to the invention, especially those which are intended to provide health benefits against cardiovascular disease, comprises vitamin B6 and vitamin B12 and folic acid.

The amount of the B-vitamins in the functional food product may be calculated by the skilled person based on daily amounts of these B-vitamins given herein: Folic acid: 200-800 $\mu$g/day, preferably 200-400 $\mu$g/day; Vitamin B6: 0.2 - 2 mg/day, preferably 05-1 mg/day and Vitamin B12: 0.5 - 4 $\mu$g/day, preferably 1 - 2 $\mu$g/day.

Preferably, the food product comprises one or more phytosterols, phytostanols and/or analogues or derivatives thereof.

Typically, the phytosterols, phytostanols and their analogues and derivatives may be selected from one or more of phytosterols, phytostanols, synthetic analogues of phytosterols and phytostanols and esterified derivatives of any of the foregoing, and mixtures of any of these. The total amount of such substances in a food product or food supplement is preferably from 0.01% to 20%, more preferably from 0.1% to 15%, still more preferably from 0.2% to 8%, and most preferably from 0.3% to 8% by weight of the food product composition.

Preferably, the daily intake of such sterol-type component of the combination is from 0.1g to 3g, more preferably from 1.5g to 2.5g, especially from 2g to 2.25g per day.

Phytosterols, also known as plant sterols or vegetable sterols can be classified in three groups, 4-desmethylsterols, 4-monomethylsterols and 4,4'-dimethylsterols. In oils they mainly exists as free sterols and sterol esters of fatty acids although sterol glucosides and acylated sterol glucosides are also present. There are three major phytosterols namely beta-sitosterol, stigmasterol and campesterol. Schematic drawings of the components meant are as given in "Influence of Processing on Sterols of Edible Vegetable Oils", S.P. Kochhar; Prog. Lipid Res. 22: pp. 161-188.

The phytostanols are the respective 5$\alpha$- saturated derivatives of phytosterols such as sitostanol, campestanol and their derivatives.

Synthetic analogues of any of the phytosterols or phytostanols (which include chemically modified natural phytosterols or phytostanols) may be used.

Preferably the phytosterol or phytostanol is selected from the group comprising fatty acid ester of ß-sitosterol, ß-sitostanol, campesterol, campestanol, stigmasterol, stigmastanol and mixtures thereof.

The optional phytosterol or phytostanol materials recited above may optionally be provided in the form of one or more fatty acid esters thereof. Mixtures of esterified and non-esterified materials may also be used.

Thus, any of the phytosterols, phytostanols and their synthetic analogues used in the present invention are preferably esterified with a fatty acid. Preferably, they are esterified with one or more $C_{2-22}$ fatty acids. For the purpose of the invention the term $C_{2-22}$ fatty acid refers to any molecule comprising a $C_{2-22}$ main chain and at least one acid group.

Although not preferred within the present context the $C_{2-22}$ main chain may contain 1-6 double bonds, be partially substituted or side chains may be present. Preferably, however the $C_{2-22}$ fatty acids are linear molecules comprising one or two acid group(s) as end group(s). Most preferred are linear $C_{8-22}$ fatty acids as occur in natural liquid oils.

Suitable examples of any such fatty acids are acetic acid, propionic acid, butyric acid, caproic acid, caprylic acid, capric acid. Other suitable acids are for example citric acid, lactic acid, oxalic acid and maleic acid. Most preferred are lauric acid, palmitic acid, stearic acid, arachidic acid, behenic acid, oleic acid, cetoleic acid, erucic acid, elaidic acid, linoleic acid and linolenic acid.

When desired a mixture of fatty acids may be used for esterification of the sterols. For example, it is possible to use a naturally occurring fat or oil as a source of the fatty acid and to carry out the esterification via an interesterification reaction. Use of a natural source nearly always results in a mixture of fatty acids.

In a particular embodiment, the fatty acid mixture contains a high amount (>50%, preferably >70%, further preferred >80%) of unsaturates, being either monounsaturated fatty acids (MUFA) and/or polyunsaturated fatty acids (PUFA). This does not only provide the advantage of e.g. PUFA itself having good blood cholesterol lowering capacity, but also of the sterols esters prepared with such fatty acids.

Preferably fatty acid mixtures of sunflower, safflower, rapeseed, linseed, olive oil, linola and/or soybean are used. These are typical sources of high PUFA and/or low SAFA. Suitable esterification conditions are for example described in WO 92/19640.

The above described food ingredients, contributing to increasing cardiovascular health, K+, Ca2+ and Mg2+, B-vitamins (folic acid, B6, B12) and sterols are herein collectively referred to as "heart health" ingredients.

Functional food products; formats

The functional food products according to the invention may be in any suitable product format. Suitable product formats include beverages, more preferably fruit juice beverages or dairy beverages optionally with added fruit juice, soy-based beverages optionally with added fruit juice, dairy-type products such as yoghurt, quark and cheese, custards, rice or other similar pudding, puddings such as mousses and other desserts including frozen confectionery products, spreads/margarines, pasta products and other cereal products, meal replacement products and other nutrition bars, sauces and dressings such as salad dressings and mayonnaise, fillings, dips, and breakfast type cereal products such as porridge. Some of these types of food products are described in some detail below and in the examples.

• Fruit juice products

Examples of fruit juice products according to the invention are juices derived from citrus fruit like orange and grapefruit, tropical fruits, banana, peach, peer, strawberry, to which Health-benefit-solid and/or Health-benefit-fraction and optionally one or more heart health ingredients, or other ingredients providing health benefits, are added.

• Dairy type products

Examples of dairy products according to the invention are milk, dairy spreads, cream cheese, milk type drinks and yoghurt, to which the health-benefit-solid and/or health-benefit-fraction, and optionally one or more heart health ingredients, or other ingredients having a health benefit are added.

The food product may be used as such as a milk type drink. Alternatively flavour or other additives may be added. A dairy type product may also be made by adding health-benefit-solid and/or health-benefit-fraction to water or to a dairy product.

An example of a composition for a yoghurt type product is about 50-80 wt.% water, 0.1-15 wt.% health-benefit-solid and optionally one or more heart health, or other health-benefit agent, ingredients, 0-15 wt.% whey powder, 0-15 wt.% sugar (e.g. sucrose), 0.01-1 wt.% yoghurt culture, 0-20 wt.% fruit, 0.05-5 wt.% vitamins and minerals, 0-2 wt.% flavour, 0-5 wt.% stabilizer (thickener or gelling agent). To the yoghurt, fruit may be added.

A typical serving size for a yoghurt type product could be from 50 to 250 g, generally from 80 to 200 g.

• Frozen Confectionery Products

For the purpose of the invention the term frozen confectionery product includes milk containing frozen confections such as ice-cream, frozen yoghurt, sherbet, sorbet, ice milk and frozen custard, water-ices, granitas and frozen fruit purees.

Preferably the level of solids in the frozen confection (e.g. sugar, fat, flavouring etc) is more than 3 wt.%, more preferred from 10 to 70 wt.%, for example 40 to 70 wt.%.

Ice cream will typically comprise 0 to 20 wt.% of fat, 0.1 to 20 wt.% health-benefit-solid and optionally one or more heart health or other health benefit ingredients, sweeteners, 0 to 10 wt.% of non-fat milk components and optional components such as emulsifiers, stabilisers, preservatives, flavouring ingredients, vitamins, minerals, etc, the balance being water. Typically ice cream will be aerated e.g. to an overrun of 20 to 400 %, more specific 40 to 200 % and frozen to a temperature of from -2 to - 200°C, more specific -10 to -30 °C. Ice cream normally comprises calcium at a level of about 0.1 wt%.

• Meal Replacement Products

Meal replacement products are preferred when the health benefit(s) to be provided includes prevention of obesity or body weight control. This health benefit may, for example, take the form of body weight reduction or body weight maintenance, appetite suppression or the provision of satiety. Such products are usually used as part of a dietary programme to control or reduce body weight.

The term "meal replacement products" as used herein includes compositions includes products which are intended to be eaten in place of a normal meal. Nutrition bars and beverages which are intended to constitute a meal replacement are types of meal replacement products. The term also includes products which are eaten as part of a meal replacement weight loss or weight control plan, for example snack products which are not intended to replace a whole meal by themselves but which may be used with other such products to replace a meal or which are otherwise intended to be used in the plan; these latter products typically have a calorie content in the range of from 50-200 kilocalories per serving.

"Enhanced feelings of satiety" as used herein means a greater or enhanced feeling of satiety (satiation) after eating and/or a longer lasting feeling of satiety after eating. Such effects typically reduce feelings of hunger and/or extend the time between food intake by an individual and can result in a smaller amount of food and/or fewer calories consumed in a single or subsequent sitting. The references herein to satiety include both what is strictly referred to as satiation and satiety, including end-of-meal satiety and between-meals satiety. Satiety may also be perceived by an individual as a feeling of 'fullness', reduced hunger and/or reduced appetite.

The meal replacement product may be in the form of a nutrition bar. The bars may be a granola-style bar ad may be based on any types of cereal. Typically the bars will be fortified with vitamins and minerals.

The meal replacement product may be in the form of a beverage product. Preferred types of beverages include powdered beverages, ready-to-drink beverages and soups. Such beverages may be dairy based, such as milk or yoghurt drinks, or may be soy based drinks.

• Other functional food products

Other functional food product according to the invention can be prepared by the skilled person based on common general knowledge comprising MAP as such or in a protein hydrolysate and optionally one or more heart health, or other health-benefit ingredients in suitable amounts. Examples of such food products are baked goods such as cakes, biscuits and muffins, dairy type foods, snacks, etc.

Beverages according to the invention may also be non-protein containing drinks such as sports-type beverages, tea based beverages, coffee based beverages or vegetable based beverages.

Advantageously the functional food product may be an oil and water containing emulsion, for instance a spread. Oil and water emulsion is herein defined as an emulsion comprising oil and water and includes oil in water (O/W) emulsions and water in oil emulsions (W/O) and more complex emulsions for instance water-in-oil-in-water (W/O/W/O/W) emulsions. Oil is herein defined as including fat. Preferably the food product is a spread, frozen confection, or sauce. Preferably a spread according to the invention comprises 30-90 wt.% vegetable oil. Advantageously a spread has a pH of 4.2-6.0.

The prevention of obesity /body weight control

We have found that the tripeptides MAP and ITP are stable in the human intestinal tract and thus this provides for the possibility of these tripeptides providing a range of valuable health-benefits to the consumer thereof. Certain whey-derived peptides are known to exhibit good effects in the prevention of obesity and/or body weight control and appear to act by regulating appetite/satiety. Obesity is generally acknowledged as being represented by a body mass index of 30 or greater.

The tripeptides MAP and ITP and/or the salts thereof are therefore believed to be very suitable for use in the prevention of obesity and/or body weight control, especially in appetite suppression and the provision of satiety.

Increased satiety leads generally to decreased food intake in a subject and can help to provide one or more of the following benefits. These benefits are generally associated with the prevention of obesity/weight control; to loose or maintain body weight, to

maintain/build lean body mass, to reduce fat mass, to reduce visceral fat, to reduce subcutaneous fat, to improve muscle tone/appearance, to maintain healthy blood sugar levels, to treat/prevent metabolic syndrome, to improve blood flow (e.g. to brain) and/or to improve blood circulation.

The person skilled in the art is well aware of how to determine such properties for a material. For example, a Suitable test for appetite suppression/satiety is given in WO 2004/002241.

Cardiovascular health maintenance

One health problem which is associated with a negative impact upon cardiovascular health maintenance is high blood pressure (hypertension) which can lead to target organ damage and different organs may be affected. In the brain, prolonged hypertension predisposes an individual to the occurrence of strokes whether by occlusion (ischemic infarct) or by bleed (hemorrhagic infarct). In addition, hypertension is related to risk of Transient Ischeamic attack (TIA) and dementia. Other consequences on the level of the brain include: headache, nausea/vomiting, anxiety, obtundation, seizures, weakness, asymmetric reflexes, facial palsy.

Hypertension increases the work needed to be done by the heart to meet the demands of the body. This prolonged increase in the workload of the heart eventually results to enlargement of the heart (in particular the left ventricle) and predisposes an affected subject to the occurrence of heart failure and heart attack / myocardial infarction. In addition to the effects on the heart, all arteries are affected by high blood pressure leading to "peripheral arterial disease".

Prolonged hypertension can also result in kidney failure / renal impairment, (related to proteinuria, heamaturia, edema and ascites) which in the end-stage may necessitate dialysis treatment. Lastly, the eyes are particularly sensitive to hypertension. Visual disturbances result from retinopathy, decreased pupillary ligth reflex, papilledema, optic atrophy, fundal haemorrages and exudate.

According to the present invention, we have found that the tripeptides MAP and ITP have a high ACE-inhibiting effect, corresponding to a low IC50 value, respectively 0.4 for MAP and 10 for ITP (in $\mu$M) as determined in the experimental part herein. Moreover, as stated above, we have found that both tripeptides MAP and ITP are stable in the human intestinal tract. The tripeptide MAP and/or the tripeptide ITP and salts thereof are therefore very suitable as an angiotensin-converting enzyme inhibitor, in particular in vivo in humans. Preferably the angiotensin-converting enzyme inhibitor is a functional food product.

The invention provides a food product suitable for angiotensin-converting enzyme inhibition, comprising an amount of 0.5 mg/kg or more of MAP and optionally 3 mg/kg or more of tripeptide ITP. Due to its ACE-inhibiting effect the food product according to the invention is capable of lowering the blood pressure of humans having elevated blood pressure, and is particularly suited to lowering blood-pressure in humans having moderately elevated blood pressure. Preferably the food product comprises an amount of 1 mg/kg or more MAP and optionally an amount of 6 mg/kg or more tripeptide ITP. More preferably the food product comprises 2 mg/kg or more MAP and optionally 12 mg or more ITP, even more, preferably 5 mg/kg to 20mg/kg or more MAP and optionally 25-100 mg/kg ITP.

Increased blood cholesterol levels lead to atherosclerosis, which results in the narrowing of arteries in various organs. The mostly affected organ is the heart and thus the control of blood cholesterol levels forms a part of maintaining good cardiovascular health. Narrowed coronary arteries cause coronary heart disease with symptoms such as angina or heart attack. In the brain, an atherosclerotic carotid or cerebral (brain) artery results in a stroke. In legs, an atherosclerotic arteries lead to leg pain or acutely ischaemic legs. In other organs (such as kidney), an atherosclerotic arteries lead to relevant ischaemic symptoms (kidney failure). According to a particular embodiment of the present invention, there is provided the use of MAP and/or to control blood cholesterol levels as this plays a part in cardiovascular health maintenance.

The present invention will be further described with reference to the following examples. Further modifications within the scope of the present invention will be apparent to the person skilled in the art.

**Examples**

**Analysis techniques: ACE-inhibiting effect**

***High Resolution Screening -mass spectrometry (HRS-MS)***

MAP and ITP as novel ACE inhibiting peptides were identified in samples by using 2-dimensional-chromatographic-separation combined with an at-line ACE activity assay and mass spectrometry for identification. In the first analysis the peptide mixture is separated on an ODS3 liquid chromatography (LC) column. An activity profile is created from fractions collected from the analysis using a slightly modified Matsui assay. In the second analysis the fractions from the first column showing a high activity are further separated on a Biosuite LC column using a different gradient profile. The fractions collected from this second column are split into two parts, one part is used for the activity measurement while MS and MS-MS is applied on the other part for identification of the peptides present.

All analyses were performed using an Alliance 2795 HPLC system (Waters, Etten-Leur, the Netherlands) equipped with a dual trace UV-detector. For identification of the peptides the HPLC-system was coupled to a Q-TOF mass spectrometer from the same supplier.

20 μl of a 10% (w/v) solution of PH Milli-Q water was injected on a 150 x 2.1 Inertsil 5 ODS3 column with a particle size of 5 μm (Varian, Middelburg, the Netherlands). Mobile phase A consisted of a 0.1% trifluoroacetic acid (TFA) solution in Milli-Q water. Mobile phase B consisted of a 0.1% TFA solution in acetonitrile. The initial eluent composition was 100% A. The eluent was kept at 100% A for 5 minutes. Then a linear gradient was started in 10 minutes to 5% B, followed by a linear gradient in 10 minutes to 30% B. The column was flushed by raising the concentration of B to 70% in 5 minutes, and was kept at 70% B for another 5 minutes. After this the eluent was reduced to 100% A in 1 minute and equilibrated for 9 minutes. The total run time was 50 minutes. The effluent flow was 0.2 ml min$^{-1}$ and the column temperature was set at 60 °C. A UV chromatogram was recorded at 215 nm. Eluent fractions were collected in a 96 well plate using a 1 minute interval time resulting in fraction volumes of 200 μl. The effluent in the wells was neutralised by addition of 80 μl of a 0.05% solution of aqueous ammonium hydroxide (25%). The solvent was evaporated until dryness under nitrogen at 50°C. After this the residue was reconstituted in 40 μl of Milli-Q water and mixed for 1 minute. Then 27 μl of a 33.4 mU ml$^{-1}$ Angiotensin Converting Enzyme (ACE) solution in phosphate buffered saline (PBS) pH 7.4 with a chloride concentration of 260 mM was added and the mixture was allowed to incubate for 5 minutes on a 96 well plate mixer at 700 RPM. After the incubation period 13 μl of a 0.35 mM hippuric acid-histidine-leucine (HHL) solution in PBS buffer was added and mixed for 1 minute at 700 RPM. The mixture was allowed to react for 60 minutes at 50 °C in a GC-oven. After the reaction the plate was cooled in melting ice and analysed on a flash-HPLC-column. 30 μl of the reaction mixture of each well was injected on a Chromlith Flash RP18e 25 x 4.6 mm HPLC column (Merck, Darmstadt, Germany) equipped with a 10 x4.6 mm RP18e guard column from the same supplier. The 5 isocratic mobile phase consisted of a 0.1% solution of TFA in water/acetonitrile 79/21. The eluent flow was 2 ml min$^{-1}$ and the column temperature was 25°C. The injections were performed with an interval time of 1 minute. Hippuric acid (H) and HHL were monitored at 280 nm. The peak heights of H and HHL were measured and the ACEI of each fraction was calculated according to the equation:

$$ACEIa = \frac{(DH_w - DH_a)}{DH_w * 100}$$

|  |  |
|---|---|
| *ACEIα* | Percentage inhibition of the analyte |
| *DH$_w$* | Degree of hydrolysis of HHL to H and HL in water |
| *DH$_a$* | Degree of hydrolysis of HHL to H and HL for the analyte |

The degree of hydrolysis (DH) was calculated by expressing the peak height of H as a fraction of the sum of the peak heights of H and HHL.

The highest activity was measured in the fractions eluting between 18 and 26 minutes. This region was collected and reinjected on a 150 x 2.1 mm Biosuite column with a particle size of 3 μm (Waters, Etten-Leur, the Netherlands). Mobile phase A here consisted of a 0.1% formic acid (FA) solution in Milli-Q water. Mobile phase B consisted of a 0.1% FA solution in methanol. The initial eluent composition was 100% A. The eluent was kept at 100% A for 5 minutes. After this a linear gradient was started in 15 minutes to 5% B, followed by a linear gradient in 30 minutes to 60% B. The eluent was kept at 60% B for another 5 minutes. Finally the eluent was reduced to 100% of mobile phase A in 1 minute and equilibrated for 10 minutes. The total run time was 65 minutes. The eluent flow was 0.2 ml min$^{-1}$ and the column temperature was set at 60°C. The UV trace was recorded at 215 nm. Fractions were collected from the Biosuite column at 10 seconds interval time. The fractions were split into two parts, one part was used to measure the activity using the ACE method described earlier, while the other part was used to identify the active peptides using MS and MS-MS.

Two chromatographic peaks with molecular ions of 326.2080 Da and two other peaks with molecular ions of 330.2029 Da and 318.1488 Da corresponded with the increased activities measured in the area between 18 and 26 minutes. Using MS-MS these peptides were identified as the structural isomers IPP and LPP (- 0.6 ppm), ITP (-4.8 ppm) and MAP (+2. 8 ppm) respectively. The protein sources of the peptides are IPP ß-casein f74-76, LPP β-casein f151-153, ITP α-s2-casein f119-121 and MAP β-casein f102-104. IPP and LPP are reported earlier as ACEI peptides with IC50 values of 5 and 9.6 μM respectively (Y. Nakamura, M. Yamamoto., K. Sakai., A. Okubo., S. Yamazaki, T. Takano, J. Dairy Sci. 78 (1995) 777-783; Y. Aryoshi, Trends in Food Science and Technol. 4 (1993) 139-144).

ITP and MAP are, to our knowledge, not earlier reported as ACEI peptides. The peptides were synthesised and the activity of each peptide was measured using a modified Matsui assay described hereafter. The IC50 values of ITP and MAP were determined to be 10 $\mu$M and 0.4 $\mu$M, respectively.

Quantification of MAP and ITP in the samples was performed on a Micromass Quattro II MS instrument operated in the positive electrospray, multiple reaction monitoring mode. The HPLC method used was similar to the one described above. The MS settings (ESI+) were as follows: cone voltage 37 V, capillary voltage 4 kV, drying gas nitrogen at 300 1/h. Source and nebulizer temperature: 100°C and 250°C, respectively. The synthesized peptides were used to prepare a calibration line using the precursor ion 318.1 and the summed product ions 227.2 and 347.2 for MAP and using the precursor ion 320.2 and the summed product ions 282.2 and 501.2 for ITP.

*ACE activity measurement of MAP and ITP using a modified Matsui assay*

This ACE inhibition activity was assayed according to the method of Matsui et al. (Matsui, T. et al. (1992) Biosci. Biotech. Biochem. 56: 517-518) with the modifications described below.

Table 1: procedure for Matsui ACE inhibition assay. The components were added in a 1.5-ml tube with a final volume of 120 $\mu$l.

| Component | Control 1 ($\mu$l) | Control 2 ($\mu$l) | Sample 1 ($\mu$l) | Sample 2 ($\mu$l) |
|---|---|---|---|---|
| HHL (3 mM) | 75 | 75 | 75 | 75 |
| H$_2$O | 25 | 45 | - | 20 |
| Sample/ inhibitor | - | - | 25 | 25 |
| ACE (0.1 U/ml) | 20 | - | 20 | - |

For each sample 75 $\mu$l 3 mM hippuryl histidine leucine (Hip-His-Leu, Sigma chemicals Co.; the chemical was dissolved in 250 mM Borate containing 200 mM NaCl, pH 8.3); 20 $\mu$l 0.1 U/ml ACE (obtained at Sigma) or H$_2$O, and 25 $\mu$l sample or H$_2$O were mixed (see Table 1). The mixtures were incubated at 37°C and stopped after 30 min by adding 125 $\mu$l 0.5 M HCl. Subsequently, 225 $\mu$l bicine/NaOH solution (1 M NaOH : 0.25 M bicine (4:6)) was added, followed by 25 $\mu$l 0. 1 M TNBS (2,4,6-Trinitrobenzenesulfonic acid, Fluka, Switzerland; in 0.1 M Na$_2$HPO$_4$). After incubation for 20 min. at 37°C, 4 ml 4 mM Na$_2$SO$_3$ in 0.2 M NaH$_2$PO$_4$ was added and the absorbance at 416 nm was measured with UV/Vis spectrophotometer (Shimadzu UV-1601 with a CPS controller, Netherlands).

The amount of ACE inhibition (ACEI) activity was calculated as a percentage of inhibition compared with the conversion rate of ACE in the absence of an inhibitor:

$$\text{ACEI (\%)} = (((C1-C2)-(S1-S2))/(C1-C2)) * 100 \qquad (1)$$

wherein
C1 = Absorbance without ACE inhibitory component (= max. ACE activity) [AU].
C2 = Absorbance without ACE inhibitory component and without ACE (background) [AU].
S1 = Absorbance in the presence of ACE and the ACE inhibitory component [AU].
S2 = Absorbance in the presence of the ACE inhibitory component, but without ACE [AU].

*HRS-MS analysis of hydrolyzed samples*

As a result, the important ACE inhibiting peptides found in PH were MAP ($\beta$-casein, pos 102-104), and ITP ($\alpha$-s2-casein, pos 119-121) at a concentration of 2.85 and 1.41 mg/g, respectively (table 1). The IC$_{50}$ of MAP and ITP were determined to be 0.4 and 10 $\mu$M, respectively.

Milk proteins and milk protein hydrolysates are commonly known as precursors of a large range of ACE inhibitory peptides. After consumption, the proteins and peptides are subjected to various digestive enzymatic processes in the human gastrointestinal tract, which results in the release of in-vivo ACE inhibitory peptides. In order to assess the break-down of the identified bioactive peptides and the formation of novel active peptides after human consumption, PH was processed by an artificial gastro-intestinal tract, which simulated conditions typically found in the human body. At certain times samples were taken from the GIT model system. These were also analysed using the on-line HPLC-Bioassay-MS or HRS-MS system.

It showed that both MAP and ITP are of particular importance because of their high resistance against GIT digestion and their high activity therefore has a very high potential to be a blood pressure lowering peptide.

**Example 1 - Identification of the novel and potent ACE inhibiting tripeptides MAP and ITP in concentrated casein hydrolysates**

To facilitate a more thorough analysis of bio-active peptides present, the casein hydrolysate obtained by the digestion with pure A. niger derived proline specific endoprotease and purified by acid precipitation was prepared on a preparative scale. To that end 3000 grams of potassium caseinate was suspended in 25 liters of water of 75 degrees C. After a thorough homogenisation the pH was slowly adjusted to 6.0 using diluted phosphoric acid. After cooling down to 55 degrees C, the A. niger derived proline specific endoproteases was added in a concentration of 4 enzyme units/ gram caseinate (see Materials & Methods section for unit definition). After an incubation (with stirring) for 3 hours at 55 degrees C, the pH was lowered to 4.5 by slowly adding concentrated phosphoric acid. In this larger scale preparation the heat treatment step to inactivate the proline specific endoprotease at this part of the process was omitted. Then the suspension was quickly cooled to 4 degrees C and kept overnight (without stirring) at this temperature. The next morning the clear upper layer was decanted and evaporated to reach a level of 40% dry matter. The latter concentrated liquid was subjected to a UHT treatment of 4 seconds at 140 degrees C and then ultrafiltered at 50 degrees C. After germ filtration, the liquid was spray dried. This material is hereinafter referred to as Casein Derived Bio-Active Peptides (CDBAP). Using the LC/MS procedures outlined in the Materials &Methods section, the IPP, LPP and VPP content of the powdered product was determined. According to its nitrogen content, the powdered product has a protein content of about 60 % (using a conversion factor of 6.38). The IPP, LPP and VPP contents of the powder are provided in Table 6. The amino acid composition of the CDBAP product is provided in Table 7. Quite remarkable is the increase of the molar proline content of the spray dried material obtained after acid precipitation: from an initial 12 % to approx 24%.

Table 2: IPP, LPP and VPP content of CDBAP.

| IPP | LPP | VPP |
|---|---|---|
| Tripeptide content in mg / gram powder | | |
| 2.5 | 6.5 | < 0.1 |
| Tripeptide content in mg / gram protein | | |
| 4.2 | 10.8 | < 0.17 |

Table 3: Amino acid composition of the potassium caseinate starting material and CDBAP (amino acid contents after acid hydrolysis and shown as percentages of the molar amino acid content).

| Amino Acid | Starting material | CDBAP |
|---|---|---|
| Asp | 6.5 | 3.2 |
| Glu | 18.9 | 12.5 |
| Asn | - | - |
| Ser | 6.7 | 4.3 |
| Gln | - | - |
| Gly | 3.5 | 3.2 |
| His | 2.2 | 3.7 |
| Arg | 2.8 | 2.3 |
| Thr | 4.3 | 3.0 |
| Ala | 4.5 | 3.4 |
| Pro | 12.3 | 24.1 |
| Tyr | 3.9 | 2.4 |
| Val | 7.1 | 9.6 |
| Met | 2.3 | 3.9 |
| Ile | 5.0 | 4.1 |

(continued)

| Amino Acid | Starting material | CDBAP |
|---|---|---|
| Leu | 9.2 | 9.0 |
| Phe | 4.0 | 3.9 |
| Lys | 6.9 | 7.4 |
| Total | 100 | 100 |

**Example 2** - Simulated *in-vitro* gastro-intestinal digestion of a hydrolyzed casein protein isolate obtained from DSM (Delft, The Netherlands).

Digestion of protein hydrolysate (hereafter PH) , a hydrolyzed casein protein isolate obtained from DSM (Delft, The Netherlands). The protein hydrolysate (PH) was prepared by incubation of 10wt% potassium caseinate with overproduced and essentially pure endoprotease from Aspergillus niger as described in WO 02/45524.

The digestion procedure was performed using a dissolution model (Vankel) with a 100 ml flask. The temperature of the water bath was set to 37.5°C and the paddle speed was chosen such that the sample was kept in suspension (100 rpm).

About 3.4 grams of PH (protein level of 59%) was dissolved / suspended in 100 ml Milli-Q water. During gastric simulation 5 M HCl was used to decrease the pH, at the end of gastric simulation and during the duodenal phase 5 M NaOH was used to raise the pH.

The protein hydrolysate suspension was preheated to 37.5°C.

At t = 0 min 0.31g of pepsin (Fluka order no. 77161) was suspended separately in 5 ml of the sample and was directly added.

The pH was adjusted slowly by hand using a separate pH meter according to the following scheme;

t = 20 min    pH decreased to 3.5
t = 40 min    pH to 3.0
t = 50 min    pH to 2.3
t = 60 min    pH to 1.8
t = 65 min    pH raised to 2.7
t = 75 min    pH to 3.7
t = 80 min    pH to 5.3

At t = 90 min 0.139 g of 8 times USP pancreatin (Sigma order no. P7545) was suspended separately in 5 ml of the sample and was directly added;

t = 93 min     pH to 5.5
t = 95 min     pH to 6.3
t = 100 min    pH to 7.1

The experiment was stopped at t = 125 min and the pH was checked (was still pH 7).

The samples were transferred into a beaker and were heated in a microwave till boiling. Subsequently, the samples were transferred into glass tubes and incubated at 95°C for 60 min. This is necessary to inactivate all protease activity. After cooling the samples were put in falcon tubes and centrifuged for 10 min at 3000 x g. The supernatant was freeze dried. The total N concentration was determined and converted to protein level using the Kjeldahl factor of casein (6.38). The protein level of the PH digest was 48.4%.

Table 4: Results of example 1. Concentration of MAP and ITP in PH and in the digested product in an artificial human gastro intestinal tract (mg/L).

| Sample | Concentration in $\mu$g g$^{-1}$ powder | |
|---|---|---|
| | MAP | ITP |
| | | |
| PH in example 1 | 2851.4 | 903.74 |

(continued)

| Sample | Concentration in $\mu g\ g^{-1}$ powder | |
|---|---|---|
| | MAP | ITP |
| | | |
| PH after digestion | 3095.8 | 889.13 |

Table 5: ACE inhibition (IC50 values) of MAP, ITP and IPP, the values determined by the at-line ACE assay and the modified Matsui assay.

| Peptide | IC50 value in $\mu M$ | |
|---|---|---|
| | At-line ACE assay | Modified Matsui assay |
| MAP | 3.8 | 0.4 |
| ITP | 50 | 10 |
| IPP (reference) | 7.1 | 2 |

**Example 3** - Simulated *in-vitro* gastro-intestinal digestion of synthetic MAP and ITP.

In order to measure stability of the peptides in the gastrointestinal tract (GI) micro-dissolution was used. This following test was used to test the GI stability of MAP and ITP.

Components:

For the dissolution the following solutions were used:

0.1 mol/l HCl
1 mol/l NaHCO3

Simulated gastric fluid;
1.0 g sodium chloride en 3.5 ml 0.1 mol/l HCl in 500 ml water (degassed in sonification bath, 10 min.)

Enzymes gastric conditions (amounts needed in 1 ml total volume) :

2.9 mg Pepsine en 0.45 mg Amano Lipase-FAP15 in 50 $\mu l$ simulated gastric fluid

Enzymes intestinal conditions (amounts needed in 1 ml total volume) :

9 mg Pancreatine (Sigma P8096) en 0.125 mg bile extract in 50 $\mu l$ 1.0 mol/l NaHCO3

Procedure:

Gastric conditions:

- Each vial was filled with:
- 0.82 ml simulated gastric fluid + 70 $\mu l$ MilliQ + 10 $\mu g$ (10 x diluted) Mixture 1,
- take a sample when T = 37.5°C (t=0), add 50 $\mu l$ pepsine/lipase mixture (shake).
- The pH is measured and adjusted to 3.5 with 0.1 mol/l HCl
- Incubation for 60 minutes, after 60' a sample is taken.

Intestinal conditions:

- 50 $\mu l$ pancreatine mixture is added, the pH is measured and adjusted to 6.8 with
- HCl.
- Samples are taken at 5', 30' en 60' after the addition of pancreatine (shake).
- All samples are kept at 95°C for 60 minutes to stop the enzyme from being active.

- After cooling the samples were stored at -20 °C until analysis.
- The samples were centrifuged and analyzed with HPLC-MRM-MS.

(For VAWWMY much more energy was needed to fragment it, in order to measure it. This was necessary because of the largeness of the peptide.)

Insel IS89 was used (gently shaking at 0), which is an incubation device meant for 96 wells plates.

For tables 6 to 9 the measured concentration of the peptide is given in ng/ml, calculated to the relative concentration of MAP.

Table 6 - Simulated *in-vitro* gastro-intestinal digestion of synthetic MAP - 1 microgram/ml

|  | a | b |  |  |  |
|---|---|---|---|---|---|
| Time (minutes) | conc | Ng/ml | % remaining trial 1 | % remaining trial 2 | % average remaining |
| 0 | - | 2962.5 | 100 | 100 | 100 |
| 30 | - | 2760 | - | 93 | 93 |
| 60 | 1902.6 | - | 64 | - | 62 |
| 65 | 1384.6 | 1654.1 | 47 | 56 | 51 |
| 75 | 2282.2 | 1608.3 | 43 | 54 | 49 |
| 90 | 730.5 | 911.6 | 25 | 31 | 28 |
| 120 | 377.2 | 503.3 | 13 | 17 | 15 |

Where - is indicated this denotes that measurements were not taken.

Table 7 - Simulated *in-vitro* gastro-intestinal digestion of synthetic MAP - 10 microgram/ml

|  | a | b |  |  |  |
|---|---|---|---|---|---|
| Time (minutes) | conc | Ng/ml | % remaining trial 1 | % remaining trial 2 | % average remaining |
| 0 | - | 82499.2 | 100 | 100 | 100 |
| 30 | 50635.6 | 76600.6 | 61 | 93 | 77 |
| 65 | 28492.5 | 33339.1 | 35 | 40 | 37 |
| 75 | 21936.4 | 21991.9 | 27 | 27 | 27 |
| 90 | 7588.3 | 10490.8 | 9 | 13 | 11 |
| 120 | 2810.6 | 2661.8 | 3 | 3 | 3 |

Where - is indicated this denotes that measurements were not taken.

Table 8 - Simulated *in-vitro* gastro-intestinal digestion of synthetic ITP - 1 microgram/ml

|  | a | b |  |  |  |
|---|---|---|---|---|---|
| Time (minutes) | conc | Ng/ml | % remaining trial 1 | % remaining trial 2 | % average remaining |
| 0 | 1325.201 | 901.297 | 100 | 100 | 100 |
| 30 | 1236.423 | 952.165 | 93 | 106 | 99 |
| 60 | 950.665 | 893.015 | 72 | 99 | 85 |
| 65 | 722.452 | 677.991 | 55 | 75 | 65 |
| 75 | 707.693 | 698.078 | 43 | 77 | 65 |

(continued)

| Time (minutes) | a conc | b Ng/ml | % remaining trial 1 | % remaining trial 2 | % average remaining |
|---|---|---|---|---|---|
| 90 | 603.143 | 704.863 | 46 | 78 | 62 |
| 120 | 701.749 | 678.751 | 53 | 75 | 64 |

Where - is indicated this denotes that measurements were not taken.

Table 9 - Simulated *in-vitro* gastro-intestinal digestion of synthetic ITP - 10 microgram/ml

| Time (minutes) | a conc | b Ng/ml | % remaining trial 1 | % remaining trial 2 | % average remaining |
|---|---|---|---|---|---|
| 0 | 11230.3 | 9388.467 | 100 | 100 | 100 |
| 30 | 8725.68 7 | 7884.828 | 78 | 84 | 81 |
| 60 | 8542.27 1 | 9951.495 | 76 | 106 | 91 |
| 65 | 6739.74 | 8504.414 | 60 | 91 | 75 |
| 75 | 7016.45 | 6052.258 | 62 | 64 | 63 |
| 90 | 7212.26 | 5660.004 | 64 | 60 | 62 |
| 120 | 5168.85 | - | 46 | - | 46 |

Where - is indicated this denotes that measurements were not taken:

These results demonstrate that the tripeptide MAP exhibits reasonably good stability under gastro-intestinal conditions especially after 1 hour under stomach conditions. However, it does undergo further degradation before reaching the end of the gut. It is believed that MAP is protected against this degradation in the presence of other peptides within the casein hydrolysate; this explains the apparent differences in stability for MAP shown in examples 2 and 3.

The results also demonstrate the excellent stability under gastro-intestinal conditions of ITP. This excellent stability compensates for the somewhat lower potency of ITP as an ACE inhibitor compared to MAP.

**Example 4** - Preparation of fermented milk containing MAP and ITP.

Preculture preparation:

Sterile skimmed milk (Yopper ex Campina, Netherlands) was inoculated for 24 hours at 37°C with 2 to 4 % of a culture of a *Lactobacillus delbruecki* subsp. *Lactis* 05-14 (deposited at the Centraal Bureau voor Schimmelculturen (CBS), Netherlands, on 26.01.2001 and having number CBS 109270) that had been stored at -80°C as a full grown culture in the above described skimmed milk, diluted with sterile 10 % glycerol to an end concentration of 6 % glycerol. The resulting product is designated as preculture.

The strain was characterized by an API50CHL strip. The strain was able to ferment D-glucose, D-fructose, D-mannose, N-acetyl glucosamine, maltose, lactose, sucrose and trehalose. According to the APILAB Plus databank (version 5.0) it was subsequently identified as *Lactobacillus delbrueckii* subsp. *lactis.* The API50CHL strip and databank are available from bioMerieux SA, 69280 Marcy- l'Etoile, France.

Fermentation:

Reconstituted milk of 4.2% MPC-80 (Campina, Netherlands), 0.5% lactose and 0.3% Lacprodan 80 (Campina, Netherlands), was pasteurised for 2 min at 80 degrees. The milk was fermented with 2 wt % of the preculture *Lactobacillus delbruecki* subsp. *Lactis* 05-14. The fermentation was performed in 150 ml jars under static conditions and performed without pH control at 40°C.

After 24 hours a sample was taken and centrifuged for 10 min at 14.000 g. The pH was 5.3 and the MAP concentration 18.3 mg/L. ITP could not be found in the fermented milk.

**Example 5** - Muffin comprising blood pressure lowering protein hydrolysate

The MAP and ITP containing compositions according to the invention can be incorporated into a variety of products including food products. To illustrate its use in a popular pastry product, the ACE inhibiting peptides were incorporated into a muffin.

A muffin batter was prepared by first combining the following dry ingredients: 500 grams of wheat flour (Reiger from Meneba, The Netherlands), 141 grams of whole egg powder, 4.7 grams of egg white powder, 35.2 grams of dextrose, 470 grams of sucrose, 2.4 grams of emulsifier (in this case Admul 5306 of Quest, The Netherlands), 4.7 grams of salt, 7 grams of sodium bicarbonate, 9.4 grams of pyrophosphate, 1.6 grams of citric acid and 3.5 grams of sorbic acid. To this the MAP and ITP containing CDBAP powder was added to reach a final concentration of 10 grams of CDBAP powder per kg of batter. Then all dry ingredients were thoroughly mixed.

To this dry mix 475 grams of water and 475 grams of vegetable oil was added and powder and liquids were mixed for 7 minutes in speed 1 of a Hobart mixer. The resulting batter was poured into muffin trays with each individual muffin mould containing approx. 50 grams of batter. The trays were baked for 23 minutes at 195-200 degrees C. The crusts of the resulting muffins were slighty darker than the crusts of reference muffins baked without CDBAP powder added. However, the consistencies of both types of muffins were identical.

On the basis of its CDBAP content, each one of the muffins thus obtained contains approx 0.5 grams of CDBAP representing approx half of the desired daily dosage of ACE inhibiting peptides for a hypertensive person.

## Claims

1. Use of the tripeptide MAP (Met-Ala-Pro) and/or salts thereof for the manufacture of a functional food product for prevention of obesity or body weight control.

2. Use of the tripeptide MAP and/or salts thereof for the manufacture of a functional food product for the cardiovascular health maintenance, wherein the cardiovascular health maintenance comprises the control of blood cholesterol levels.

3. Functional food product capable of providing a health benefit to the consumer thereof, said health benefit selected from the prevention of obesity, body weight control and cardiovascular health maintenance and comprising an amount of 0.1 mg/kg or more of MAP and optionally 1 mg/kg or more of the tripeptide ITP.

4. Functional food product according to claim 3 wherein the cardiovascular health maintenance benefit comprises inhibition of angiotensin-converting enzyme and/or the control of blood cholesterol levels.

5. Functional food product according to either one of claims 3 or 4, wherein the amount of MAP is 0.5 mg/kg or more and optionally the amount of tripeptide ITP is 3 mg/kg or more.

6. Functional food product according to claim 5, wherein the amount of MAP is 1 mg/kg or more and optionally the amount of tripeptide ITP is 6 mg/kg or more.

7. Functional food product according to claim 6, comprising an amount of 2 mg/kg or more MAP and optionally 12 mg or more ITP.

8. Functional food product according to claim 7, comprising an amount of 5 mg/kg to 20mg/kg or more MAP and optionally 25-100 mg/kg ITP.

9. Functional food product according to any of claims 4-8, comprising 50-200 mmol/kg K+ and/or 15-60 mmol/kg Ca2+ and/or 6-25 mmol/kg Mg2+.

10. Functional food product according to claim 9, 110-135 mmol/kg K+ and/or 35-45 mmol/kg Ca$^+$ and/or 13-20 mmol/kg Mg2+.

11. Functional food product according to any of claims 3-10, comprising one or more B-vitamins.

12. Functional food product according to claim 11, comprising folic acid, vitamin B6 and vitamin B12.

13. Functional food product according to any of claim 3-12, comprising 3 to 25 wt% sterol, more preferably from 7 to 15 wt% sterol.

14. A process for preparing a functional food product, the process comprising the steps:

(a) enzymatic hydrolysis of a casein protein substrate comprising beta-casein and/or alpha-s2-casein resulting in a hydrolysed casein product;
(b) separation from the hydrolysed casein product of a fraction rich in tripeptide MAP; and
(c) using the fraction rich in tripeptide MAP as an ingredient in the preparation of the functional food product; wherein the fraction rich in tripeptide MAP from step b) is dried to obtain a solid rich in tripeptide MAP and the solid is used in step c) as an ingredient in the preparation of the functional food product.

**Patentansprüche**

1. Verwendung des Tripeptids MAP (Met-Ala-Pro) und/oder Salzen davon für die Herstellung eines Functional Food-Produkts zur Prävention von Adipositas oder zur Körpergewichtskontrolle.

2. Verwendung des Tripeptids MAP und/oder Salzen davon für die Herstellung eines Functional Food-Produkts für die Aufrechterhaltung der kardiovaskulären Gesundheit, wobei die Aufrechterhaltung der kardiovaskulären Gesundheit die Kontrolle von Blutcholesterin-Leveln umfasst.

3. Functional Food-Produkt, das geeignet ist einen Gesundheitsnutzen für den Konsumenten desselben bereitzustellen, wobei der Gesundheitsnutzen ausgewählt ist aus Prävention von Adipositas, Körpergewichtskontrolle und Aufrechterhaltung der kardiovaskulären Gesundheit, und eine Menge von 0,1 mg/kg oder mehr MAP und gegebenenfalls 1 mg/kg oder mehr des Tripeptids ITP umfasst.

4. Functional Food-Produkt nach Anspruch 3, wobei der Nutzen der Aufrechterhaltung der kardiovaskulären Gesundheit eine Inhibierung von Angiotensin umwandelndem Enzym und/oder die Kontrolle von Blutcholesterin-Leveln umfasst.

5. Functional Food-Produkt nach einem der Ansprüche 3 oder 4, wobei die Menge an MAP 0,5 mg/kg oder mehr und gegebenenfalls die Menge an Tripeptid ITP 3 mg/kg oder mehr umfasst.

6. Functional Food-Produkt nach Anspruch 5, wobei die Menge an MAP 1 mg/kg oder mehr und gegebenenfalls die Menge an Tripeptid ITP 6 mg/kg oder mehr umfasst.

7. Functional Food-Produkt nach Anspruch 6, das eine Menge von 2 mg/kg oder mehr MAP und gegebenenfalls 12 mg oder mehr ITP umfasst.

8. Functional Food-Produkt nach Anspruch 7, das eine Menge von 5 mg/kg bis 20 mg/kg oder mehr MAP und gegebenenfalls 25-100 mg/kg ITP umfasst.

9. Functional Food-Produkt nach einem der Ansprüche 4-8, das 50-200 mmol/kg $K^+$ und/oder 15-60 mmol/kg $Ca^{2+}$ und/oder 6-25 mmol/kg $Mg^{2+}$ umfasst.

10. Functional Food-Produkt nach Anspruch 9, 110-135 mmol/kg $K^+$ und/oder 35-45 mmol/kg $Ca^{2+}$ und/oder 13-20 mmol/kg $Mg^{2+}$.

11. Functional Food-Produkt nach einem der Ansprüche 3-10, das ein oder mehrere B-Vitamine umfasst.

12. Functional Food-Produkt nach Anspruch 11, das Folsäure, Vitamin B6 und Vitamin B12 umfasst.

13. Functional Food-Produkt nach einem der Ansprüche 3-12, das 3 bis 25 Gewichts-% Sterol, bevorzugter 7 bis 15 Gewichts-% Sterol, umfasst.

14. Verfahren zur Herstellung eines Functional Food-Produkts, wobei das Verfahren die Schritte:

(a) enzymatische Hydrolyse eines Caseinproteinsubstrats, das beta-Casein und/oder alpha-s2-Casein umfasst, was in einem hydrolysierten Casein-Produkt resultiert;
(b) Abtrennen einer Fraktion, die reich an Tripeptid MAP ist, aus dem hydrolysierten Casein-Produkt und
(c) Verwenden der Fraktion, die reich an Tripeptid MAP ist, als Ingrediens bei der Herstellung des Functional Food-Produkts
umfasst, wobei die Fraktion, die reich an Tripeptid MAP ist, aus Schritt b) getrocknet wird, um einen Feststoff zu erhalten, der reich an Tripeptid MAP ist, und der Feststoff in Schritt c) als Ingrediens bei der Herstellung des Functional Food-Produkts verwendet wird.

**Revendications**

1. Utilisation du tripeptide MAP (Met-Ala-Pro) et/ou d'un de ses sels pour la fabrication d'un produit alimentaire fonctionnel destiné à la prévention de l'obésité ou au contrôle de poids corporel.

2. Utilisation du tripeptide MAP et/ou d'un de ses sels pour la fabrication d'un produit alimentaire fonctionnel destiné au maintien de la santé cardiovasculaire, ledit maintien de la santé cardiovasculaire comprenant la maîtrise des taux de cholestérol sanguins.

3. Produit alimentaire fonctionnel capable d'apporter un bienfait pour la santé de celui qui le consomme, ledit bienfait pour la santé étant choisi parmi la prévention de l'obésité, le contrôle pondéral et le maintien de la santé cardiovasculaire, et comprenant une quantité de 0,1 mg/kg ou plus de MAP et éventuellement, 1 mg/kg ou plus du tripeptide ITP.

4. Produit alimentaire fonctionnel selon la revendication 3, dans lequel le bienfait du maintien de la santé cardiovasculaire comprend l'inhibition de l'enzyme de conversion de l'angiotensine et/ou la maîtrise des taux de cholestérol sanguins.

5. Produit alimentaire fonctionnel selon l'une ou l'autre des revendications 3 ou 4, dans lequel la quantité de MAP est de 0,5 mg/kg ou plus et éventuellement, la quantité de tripeptide ITP est de 3 mg/kg ou plus.

6. Produit alimentaire fonctionnel selon la revendication 5, dans lequel la quantité de MAP est de 1 mg/kg ou plus et éventuellement, la quantité de tripeptide ITP est de 6 mg/kg ou plus.

7. Produit alimentaire fonctionnel selon la revendication 6, comprenant une quantité de 2 mg/kg ou plus de MAP et éventuellement, 12 mg ou plus d'ITP.

8. Produit alimentaire fonctionnel selon la revendication 7, comprenant une quantité de 5 à 20 mg/ kg ou plus de MAP et éventuellement, 25 à 100 mg/ kg d'ITP.

9. Produit alimentaire fonctionnel selon l'une quelconque des revendications 4 à 8, comprenant 50 à 200 mmol/kg de K+ et/ou 15 à 60 mmol/kg de Ca2+ et/ou 6 à 25 mmol/kg de Mg2+.

10. Produit alimentaire fonctionnel selon la revendication 9, comprenant 110 à 135 mmol/kg de K+ et/ou 35 à 45 mmol/kg de Ca2+ et/ou 13 à 20 mmol/ kg de Mg2+.

11. Produit alimentaire fonctionnel selon l'une quelconque des revendications 3 à 10, comprenant une ou plusieurs vitamines B.

12. Produit alimentaire fonctionnel selon la revendication 11, comprenant de l'acide folique, de la vitamine B6 et de la vitamine B12.

13. Produit alimentaire fonctionnel selon l'une quelconque des revendications 3 à 12, comprenant 3 à 25 % en poids de stérol, plus préférablement, 7 à 15 % en poids de stérol.

14. Procédé de préparation d'un produit alimentaire fonctionnel, le procédé comprenant les étapes suivantes :

(a) hydrolyse enzymatique d'un substrat de protéine caséique comprenant une bêta-caséine et/ou l'alpha-s2-caséine pour obtenir un produit de caséine hydrolysé ;

(b) séparation à partir du produit de caséine hydrolysé d'une fraction riche en tripeptide MAP ; et

(c) utilisation de la fraction riche en tripeptide MAP en tant qu'ingrédient dans la préparation du produit alimentaire fonctionnel ;

dans lequel la fraction riche en tripeptide MAP provenant de l'étape b) est déshydratée pour obtenir un solide riche en tripeptide MAP et le solide est utilisé à l'étape c) en tant qu'ingrédient dans la préparation du produit alimentaire fonctionnel.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1690869 A
- WO 03102905 A
- WO 0042066 A
- WO 9219640 A
- WO 2004002241 A
- WO 0245524 A

### Non-patent literature cited in the description

- **Gerstein et al.** *The Lancet,* 2000, vol. 355, 253-259
- **Hata, Y et al.** *American Journal of Clinical Nutrition,* 1999, vol. 64, 767-771
- **Yamamoto N et al.** *Die Nahrung,* vol. 43 (3), 159-164
- **Yamamoto N et al.** *J. of Dairy Sci.,* 1994, vol. 77, 917-922
- **Maeno et al.** *J. of Dairy Sci.,* 1996, vol. 79 (8), 1316-1321
- **Y. Poilot et al.** *Journal of Membrane Science,* 1999, vol. 158, 105-114
- **S.P. Kochhar.** *Prog. Lipid Res.,* vol. 22, 161-188
- **Y. Nakamura ; M. Yamamoto. ; K. Sakai. ; A. Okubo. ; S. Yamazaki ; T. Takano.** *J. Dairy Sci.,* 1995, vol. 78, 777-783
- **Y. Aryoshi.** *Trends in Food Science and Technol.,* 1993, vol. 4, 139-144
- **Matsui, T. et al.** *Biosci. Biotech. Biochem.,* 1992, vol. 56, 517-518